# EUROPEAN PATENT APPLICATION

(11) **EP 1 072 633 A1**
(43) Date of publication of application: **31.01.2001**
(21) Application number: 99401849.7
(22) Date of filing: 22.07.1999
(51) Int. Cl.: C08J 5/18, C08L 5/00, A61K 9/48

(54) **Pullulan film compositions**

(71) Applicant: WARNER-LAMBERT COMPANY, Morris Plains New Jersey 07950 (US)
(72) Inventor: Scott, Robert, 9100 Sint Niklaas (BE); Cade, Dominique, 68000 Colmar (FR); Xiongwei, He, 68280 Andolsheim (FR)
(74) Representative: Tesch, Rudolf, Dr.

(57) **Abstract**

The invention concerns compositions base on pullulan and a setting system for the use in pharmaceutical, veterinary, food, cosmetic or other products like films for wrapping food, aspics or jellies, preferably for predosed formulations like soft or hard capsules. By using aqueous solution of the inventive compositions, the hard pullulan capsules are produced by a conventional dipping moulding process under the same process condition range than conventional gelatine capsules.

## Description

The invention concerns pullulan compositions for the use in pharmaceutical, veterinary, food, cosmetic or other products like films for wrapping food, aspics or jellies, preferably for predosed formulations like soft or hard capsules.

A second embodiment of the invention is the use of the film compositions for the manufacturing of hard capsules by conventional dip moulding process as normally used in the production of conventional hard gelatine capsules.

For the industrial manufacture of pharmaceutical capsules gelatine is most preferred for its gelling, film forming and surface active properties. The manufacture of hard gelatine capsules by dip moulding process exploits fully its gelling and film forming abilities. Such capsules are manufactured by dipping mould pins into a hot solution of gelatine, removing the pins from the gelatine solution, allowing the gelatine solution attached on pins to set by cooling, drying and stripping the so-formed shells from the pins. The setting of the solution on the mould pins after dipping is the critical step to obtain a uniform thickness of the capsule shell.

Attempts have been made to manufacture capsules with materials other than gelatine, notably with modified cellulose. Successful industrial examples are the capsules made of hydroxypropyl methylcellulose (HPMC).

Pullulan is a viscous polysaccharide extracellularly produced by growing certain yeasts on starch syrups. Its existence was reported for the first time in 1938. Hayashibara Company started the commercial production in 1976.

There are hundreds of patents about the use of pullulan such as moulded articles (FR 2147112), edible film (US 4562020) and coating (JP 02000205 A and JP 60084215 A). However there is no attempt yet of hard capsules in pullulan produced by conventional dipping moulding process

Surprisingly, we found that the addition of a very small amount of a setting system, preferably consisting of hydrocolloids, most preferably polysaccharides, confers an appropriate setting ability with cooling to pullulan solution so that the production of hard pullulan capsules can be produced with a conventional dip moulding process.

Comparing to gelatine or HPMC, the advantages of pullulan can be mentioned as follows:
- Non-animal origin
- No chemical modification, totally natural.
- Higher product quality consistency by the fermentation process control.
- High homogeneity and transparency of films
- Very low oxygen permeability. Its capsules are particularly useful for the filling of oxygen sensitive products such as fish and vegetable oils.
- Relatively low water content, lower than gelatine.
- High stability of various properties over storage such as mechanical and dissolution properties.

The aim of the invention is therefore the provision of compositions for the use in pharmaceutical, veterinary, food, cosmetic or other products like films for wrapping food, aspics or jellies, preferably for predosed formulations like soft or hard capsules and wherein the pullulan compositions has in aqueous solution a sufficient setting ability.

The addition of a setting system, preferably based on polysaccharides, to pullulan solutions enables the adaptation of specific and desired gelling properties for the production of hard pullulan capsules by a conventional dipping process. For the production of such capsules it is extremely important that the film forming pullulan solution remaining on the mould pins after dipping is prohibited from flowing down the pins. Otherwise the obtained film will not have the desired uniform thickness.

Consequently the present patent makes that the hard pullulan capsules can be produced with the same equipment used for the production of conventional hard gelatine capsules in the same range of process conditions. Furthermore capsules produced from compositions of the instant invention have the same dimensional specifications and allow the use of the existing filling machinery and do not require specific and new equipment for the filling process.

The concentration in pullulan of the dipping aqueous solution is in a range of 10 to 50%, preferably in the range of 15 to 40% by weight.

Although pullulan of various molecular weight is usable, pullulan has a viscosity from 100 cps to 2000 cps at above mentioned concentration and at dipping temperature (40-70°C) is preferred.

The pullulan without desalting (Japanese food grade) is usable, however the desalted pullulan (Japanese pharmaceutical excipients grade) is preferable for its improved mechanical properties.

The setting system consists of a hydrocolloid or mixtures of hydrocolloids and may contain in addition cations and/or sequestering agents.

Suitable hydrocolloids or mixtures producing synergistic properties may be selected from natural seaweeds, natural seed gums, natural plant exudates, natural fruit extracts, biosynthetic gums, gelatines, biosynthetic processed starch or cellulosic materials, preferred are the polysaccharides.

The preferred polysaccharides are alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, funoran, and other exocellular polysaccharides. Preferred are exocellular polysaccharides.

The preferred exocellular polysaccharides are xanthan, acetan, gellan, welan, rhamsan, furcelleran, succinoglycan, scleroglycan, schizophyllan, tamarind gum, curdlan, and dextran.

The preferred hydrocolloids are kappa-carrageenan or gellan gum or combinations like xanthan with locust bean gum or xanthan with konjac mannan.

Among the setting systems mentioned above, the systems of kappa-carrageenan with cation and gellan gum with cation are specifically preferred. They produce high gel strength at low concentrations and have good compatibility with pullulan.

The amount of the hydrocolloid is preferably in the range of 0.01 to 5% by weight and especially preferred 0.03 to 1.0% in the aqueous pullulan solution.

The cations are preferably selected from K⁺, Na⁺, Li⁺, NH₄⁺, Ca⁺⁺ or Mg⁺⁺. The amount of cations is preferably less than 3%, especially 0.01 to 1% by weight in the aqueous pullulan solution.

The preferred sequestering agents are ethylenediaminetetraacetic acid, acetic acid, boric acid, citric acid, edetic acid, gluconic acid, lactic acid, phosphoric acid, tartaric acid or salts thereof, methaphosphates, dihydroxyethylglycine, lecithin or beta cyclodextrin and combinations thereof. Especially preferred is ethylenediaminetetraacetic acid or salts thereof or citric acid or salts thereof. The amount is preferably less than 3%, especially 0.01 to 1% by weight of the dipping solution.

The inventive pullulan compositions may contain in a further aspect additional pharmaceutically or food acceptable colouring agents in the range of from 0 to 10% based upon the weight of the film. The colouring agents may be selected from azo-, quinophthalone-, triphenylmethane-, xanthene- or indigoid dyes, iron oxides or hydroxides, titanium dioxide or natural dyes or mixtures thereof. Examples are patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, brilliant black BN, carbon black, iron oxide black, iron oxide red, iron oxide yellow, titanium dioxide, riboflavin, carotenes, anthocyanines, turmeric, cochineal extract, clorophyllin, canthaxanthin, caramel, or betanin.

The inventive pullulan compositions may contain in a further aspect additional pharmaceutically or food acceptable plasticiser or flavoring agent.

The pullulan capsules of the invention may be coated with a suitable coating agent like cellulose acetate phthalate, polyvinyl acetate phthalate, methacrylic acid gelatines, hypromellose phthalate, hydroxypropylmethyl cellulose phthalate, hydroxyalkyl methyl cellulose phthalates, hydroxypropyl methylcellulose acetate succinate or mixtures thereof to provide e.g. enteric properties.

The pullulan capsules of the invention may be used for the production of containers for providing unit dosage forms for example for agrochemicals, seeds, herbs, foodstuffs, dyestuffs, pharmaceuticals, flavouring agents and the like.

The following examples and tests demonstrate the pullulan capsule production and properties:

### Example 1:

1.0 kg of pullulan (PI-20, Japanese Pharmaceutical Excipients grade) powder is mixed with 10 g of kappa-carrageenan. To 4.0 kg of deionised water under stirring at room temperature is added 20 g of potassium acetate (0.2% by weight in the solution), followed by addition of the above mixture (20% of pullulan and 0.2% of carrageenan in the solution). The powder addition and stirring speeds should be very high in order to avoid the forming of lumps, which take a long time to be dissolved. Heat the solution up to 70°C under stirring to totally dissolve the carrageenan and pullulan. It is possible to dissolve the components directly at 70°C, but the tendency of pullulan to lump is much higher.

The pullulan solution thus prepared is defoamed under slow stirring and then poured into a dipping dish of a pilot machine of conventional hard gelatine capsule production equipment. While keeping the dipping pullulan solution at 60°C, natural transparent hard pullulan capsules of size 0 were produced according to the conventional process with the same dimensional specifications to the conventional hard gelatine capsules.

### Example 2:

1.0 kg of pullulan (PI-20) powder is mixed with 6 g of gellan. To 4.0 kg of deionised water under stirring at room temperature is added 20 g of potassium acetate (0.4% by weight in the solution) and 2 g of ethylenediaminetetraacetic acid disodium salt (0.04% in the solution), followed by addition of the above mixture (20% of pullulan and 0.12% of gellan in the solution). Heat the solution up to 75°C under stirring to dissolve totally the gellan and pullulan.

The pullulan solution thus prepared is defoamed under slow stirring and then poured into a dipping dish of a pilot machine of conventional hard gelatine capsule production equipment. While keeping the dipping pullulan solution at 60°C, natural transparent hard capsules of size 0 were produced according to the conventional process with the same dimensional specifications to the conventional hard gelatine capsules.

### Disintegration test results:

Disintegration test results (according to USP XXIII 1995-<701> Disintegration):

| Capsule | Example 1 | Example 2 |
|---|---|---|
| Capsule emptied time | 3.0 min | 2.0 min |
| Total disintegration time | 10.0 min | 11.8 min |

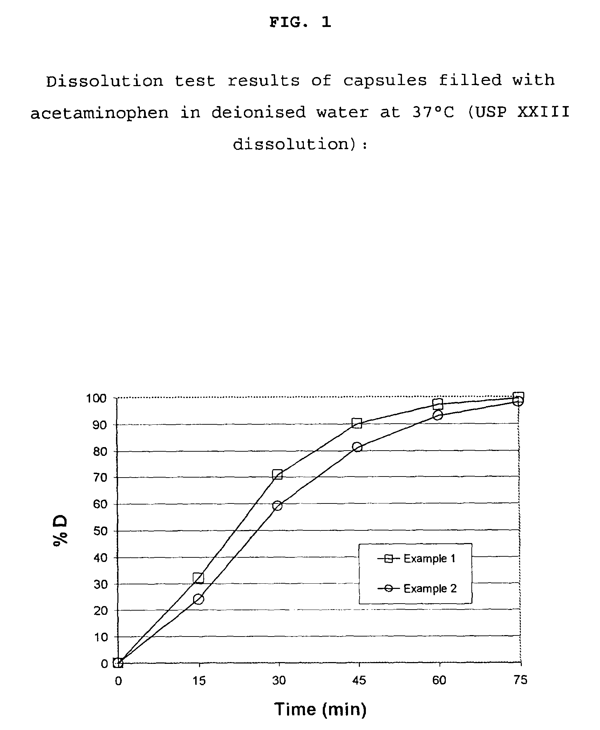

Dissolution test results of capsules filled with acetaminophen in deionised water at 37°C (USP XXIII dissolution) are represented in Fig. 1.

## Claims

1. Film forming compositions consisting of pullulan and a setting system.

2. Film forming compositions according to claim 1, wherein the setting system consists of hydrocolloids.

3. Film forming compositions according to claim 1, wherein the setting system contains optionally cations and sequestering agents.

4. Film forming compositions according to claim 1 to 3, wherein the content of pullulan is 85 to 95% by weight, of water is 5 t0 15% by weight, of polysaccharides is 0.01 to 10%, preferably 0.05 to 5% by weight, and of cation and sequestering agent is less than 5%, preferably 0.01 to 3% by weight.

5. Film forming compositions according to claim 1 and 2, wherein the hydrocolloids of the setting system are selected from polysaccharides.

6. Film forming compositions according to claim 1 and 2, wherein the hydrocolloids of the setting system are selected from alginates, agar gum, guar gum, locust bean gum (carob), carrageenan, tara gum, gum arabic, ghatti gum, Khaya grandifolia gum, tragacanth gum, karaya gum, pectin, arabian (araban), xanthan, gellan, starch, Konjac mannan, galactomannan, or funoran.

7. Film forming compositions according to claim 1 and 2, wherein the hydrocolloids of the setting system are selected from exocellular polysaccharides.

8. Film forming compositions according to claim 1 and 2, wherein the hydrocolloids of the setting system are selected from xanthan, acetan, gellan, welan, rhamsan, furcelleran, succinoglycan, scleroglycan, schizophyllan, tamarind gum, curdlan, or dextran.

9. Film forming compositions according to claim 1 and 2, wherein the hydrocolloids of the setting system are selected from gellan gum or kappa-carrageenan.

10. Film forming compositions according to claim 1 and 3, wherein the optional cations are preferably selected from K⁺, Na⁺, Li⁺, NH₄⁺, Ca⁺⁺ or Mg⁺⁺.

11. Film forming compositions according to claim 1 and 3, wherein the optional sequestering agents or mixture of sequestering agents are selected from ethylenediaminetetraacetic acid, acetic acid, boric acid, citric acid, edetic acid, gluconic acid, lactic acid, phosphoric acid, tartaric acid or salts thereof, methaphosphates, dihydroxyethylglycine, lecithin or beta cyclodextrin.

12. Film forming compositions according to claim 11, wherein the sequestering agent or mixture of sequestering agents is selected from ethylenediaminetetraacetic acid or salts thereof or citric acid or salts thereof.

13. Film forming compositions according to claims 1 to 12 containing additionally plasticizers or flavoring agents

14. Film forming compositions according to claims 1 to 13 containing additionally colouring agents in a range from about 0 to 10 % based upon the weight of the composition.

15. Film forming compositions according to claim 14 wherein the colouring agent or mixture of colouring agents is selected from azo-, quinophthalone-, triphenylmethane-, xanthene- or indigoid dyes, iron oxides or hydroxides, titanium dioxide or natural dyes.

16. Film forming compositions according to claim 15 wherein the colouring agent or mixture of colouring agents is selected from patent blue V, acid brilliant green BS, red 2G, azorubine, ponceau 4R, amaranth, D+C red 33, D+C red 22, D+C red 26, D+C red 28, D+C yellow 10, yellow 2 G, FD+C yellow 5, FD+C yellow 6, FD+C red 3, FD+C red 40, FD+C blue 1, FD+C blue 2, FD+C green 3, or brilliant black BN.

17. Film forming compositions according to claim 14 wherein the colouring agent or mixture of colouring agents is selected from carbon black, iron oxide black, iron oxide red, iron oxide yellow, titanium dioxide, riboflavin, carotenes, anthocyanines, turmeric, cochineal extract, clorophyllin, canthaxanthin, caramel, or betanin.

18. Containers for unit dosage forms for agrochemicals, seeds, herbs, foodstuffs, dyestuffs, pharmaceuticals, or flavouring agents produced from the compositions according to claims 1 to 17.

19. Container according to claim 18 which is a pharmaceutical capsule.

20. Containers according to claims 18 or 19, characterised in that they have a coating.

21. Coated containers according to claim 20 wherein the coating is selected from cellulose acetate phthalate, polyvinyl acetate phthalate, methacrylic acid gelatines, hypromellose phthalate, hydroxypropylmethyl cellulose phthalate hydroxyalkyl methyl cellulose phthalates, hydroxypropyl methylcellulose acetate succinate or mixtures thereof.

22. Caplets encapsulated in film forming compositions according to claims 1 to 17.

23. Capsules according to claim 18 or 19 characterised in that the capsule halves are sealed with one or more layers of the composition according to claims 1 to 18.

24. Capsules according to claim 18 or 19 characterised in that a liquid fusion process seals the capsule halves.

25. Capsules according to claim 18 or 19 characterised by a release of filled product at low temperature, such as at room temperature.

26. Aqueous solutions of compositions according to claims 1 to 17 for the manufacturing of capsules.

27. Aqueous solutions according to claim 26, containing pullulan in an amount of 10 to 60 %, preferably 15 to 40 % by weight, setting agent in an amount of 0.01 to 5 %, preferably 0.03 to 1.0 % by weight of the aqueous solution.

28. Aqueous solutions according to claim 26 or 27, containing optionally cations or sequestering agents in an amount less than 3 %, preferably 0.01 to 1 % by weight of the aqueous solution.

29. Use of aqueous solutions according to claims 26 to 28 for the manufacturing of hard capsules in a dip moulding process.

30. Manufacturing of hard capsules from aqueous pullulan solutions according to claims 26 to 29 in a dip moulding process with conventional hard gelatine capsules process parameters and equipment.
